# EUROPEAN PATENT SPECIFICATION

(11) **EP 2 642 957 B1**
(45) Date of publication and mention of the grant of the patent: **06.07.2016**
(21) Application number: 11807972.2
(22) Date of filing: 25.11.2011
(51) Int. Cl.: A61F 5/44, A61F 13/82, A61F 13/475, A61B 10/00

(54) **URINE SHIELD**
ÉCRAN PROTECTEUR CONTRE L'URINE
URINSCHILD

(30) Priority: 27.11.2010 GB 201020141; 01.06.2011 GB 201109205
(43) Date of publication of application: 02.10.2013
(73) Proprietor: Davidson, Angela, Sedgefield, Teeside TS21 3BP (GB)
(72) Inventor: Davidson, Angela, Sedgefield, Teeside TS21 3BP (GB)
(74) Representative: Elsworth, Dominic Stephen
(86) International application number: PCT/GB2011/052324
(87) International publication number: WO 2012/069848

(56) References cited:
- US-A1- 2005 240 164
- US-A1- 2010 152 692
- US-B1- 6 491 673

## Description

### Field of the Invention

The present invention relates to a urine shield, more particularly a shield for urine of small children.

### Background

Babies, infants and children in particular often wear nappies or diapers. During changing of the diaper there is a period during which the infant's genitalia are exposed, meaning that the infant is able to urinate freely.

Frequently due to nappy rash it may be necessary to air an infant's bottom, by leaving off the diaper for a longer period, again meaning that the infant is able to urinate freely.

An infant's diaper may also be removed during medical examinations or procedures.

This uncontrolled urination is liable to cause problems for the infant and the infant's carer and can cause soiling of both the carer's and the infant's clothing, carpets and the surrounding area.

It is of particular importance in these situations that the chances of uncontrolled urination are minimised.

United States granted patent US 7 648 489 (KRUGER) discloses a diaper changing system, relating to preventing urine exposure from urine generated by at least one infant while still permitting sufficient access to bottom portions of such at least one infant for diaper-changing purposes by at least one diaper-changer, said diaper changing system comprising: a) at least one mat structured and arranged to lay at least under thigh portions, buttock portions, and back portions of such at least one infant; b) said at least one mat having i) a top, a bottom, and an outer periphery, and ii) an upper portion and a lower portion of essentially equal dimensions, said upper portion and said lower portion essentially separated by a first line passing through the midpoint of a second line extending from said top to said bottom, said first line being perpendicular to said second line; c) at least one shield structured and arranged to lay over only the lap portion of such at least one infant by being predominantly positioned below said first line; d) wherein said at least one shield comprises portions structured and arranged to drape over the lap portion of such at least one infant; and e) at least one connector structured and arranged to connect said at least one mat to said at least one shield; f) wherein said at least one connector is positioned at said outer periphery of said at least one mat; g) at least one slit positioned adjacent the connection point of said at least one connector, said at least one slit permitting raising and lowering of said at least one shield relative to said at least one mat; h) wherein said at least one shield is structured and arranged to prevent urine exposure from urine generated by such at least one infant; and i) wherein said at least one mat and said at least one shield comprise at least one pathway structured and arranged to permit access by such at least one diaper-changer to bottom portions of such at least one infant during diaper-changing. This system is complex, and requires a user to be in possession of the whole.

United States patent application US 2007 149 935 (DIRICO) discloses a penis cap for use during a diaper change, comprising: a laminated, leak resistant bulbous shroud having a proximal end and a distal end; a substantially frustoconical base being disposed at the distal end; the frustoconical base having sidewalls forming a narrow, substantially circular elastic opening, said sidewalls being formed from an elastic material; a broad, contiguous enclosure being disposed at the proximal end so that the shroud forms a urine containment cavity; the shroud being sized to snugly fit over a baby's penis via the circular elastic opening at the base; the shroud having a substantially water proof external layer and a substantially absorptive inner layer; the shroud further comprising an intermediate buffer layer being disposed between said external layer and said absorptive inner layer, said intermediate buffer layer being adhesively joined to said external layer and said absorptive layer; and, wherein the baby, care provider and surrounding environment are protected from urine spray during a baby urination event. This cap is constrained in that either the cap fits snugly to all penises and risks discomfort on some, or a variety of sizes must be produced. This cap is likely to be difficult to use.

United States patent application US 6 080 139 (GALLEGOS) discloses an apparatus for protecting care providers from baby urination accidents comprising: a generally domed shaped, contiguous, absorbent shell terminating in a generally oblong-shaped circular base wherein the shell and base are sized such that the genitalia of a baby is accommodated and covered in the immediately surrounding region, wherein the shell includes an inner layer and an outer layer, said outer layer comprising a moisture resistant material, wherein the base carries an adhesive, wherein the apparatus is provided with a centre of gravity proximate to the baby when disposed over the genitalia thereof and wherein the base is weighted. The circular profile and domed shape of this device provides a large footprint on the infant that inhibits his movement, particularly of the infant's legs, and hence provides irritation value for the infant.

The capturing of urine from an infant for analysis for clinical or research reasons can also be problematic. Current non-invasive methods rely on either 'clean-catch' or 'pad' or 'bag' methods to collect urine. The preferred 'clean-catch' method involves catching a sample by holding a sterile specimen bottle in the urine stream. This can be unpredictable, time consuming and messy.

Alternative methods use urine collection bags and urine collection pads which are typically placed inside a diaper. The samples obtained using these methods are more susceptible to contamination than the clean-catch method, due to the close and prolonged contact with the skin around the infant's genital area.

US2010/152692 describes a female hygiene product having fluid activated barriers, that is side walls rotate into a fluid blocking position when a liquid shrinkable string connected thereto comes into contact with liquid.

US2005/240164 describes a urine collection bag for collecting urine from small children.

It would be desirable to provide an improved urine shield that can be used for protecting against an infant's urine when changing diapers or nappies or during medical procedures.

It would also be desirable to provide an improved means for collecting a urine sample from an infant.

### Summary of the Invention

According to the present invention there is provided a urine shield comprising an elongate base and side walls connected to the base, at least one of said side walls and base being absorbent or supporting an absorbent element, the shield further comprising at least one attachment tab for releasably attaching the shield to a user's skin, wherein the shield has an hourglass shaped cross-section.

At least one of said side walls and base may be absorbent. Alternatively, at least one of said side walls and base may support an absorbent element. An absorbent element may be a pad.

The shield has a front end and a rear end. The front end is for positioning close to the user's stomach area and the rear end is for positing underneath the genital area. The width of the shield at the front end and at the rear end is greater than in the centre of the shield, giving the shield the hourglass shaped cross-section. This shape allows the user greater freedom of movement and reduces irritation since, in use, the narrower part is preferably located between the user's legs.

Preferably, both the base and the side walls are absorbent. Preferably the inside of the shield is lined with a highly absorbent material.

The elongate profile of the shield increases the surface area of the shield, which in turn increases the amount of urine that can be soaked up.

Preferably at least one of the base and sides is provided with a urine indicator that changes colour when exposed to urine. Favourably the shield is provided with a printed design. This design preferably alters in colour to show urine. Furthermore the pattern on the shield may be moisture activated, therefore, in contact with urine, colour would appear on the pattern, alerting a carer.

Preferably the at least one attachment tab is attached to the side walls. Preferably at least two attachment tabs are attached to the side walls. More preferably the tabs are attached to the side walls at the front end of the shield.

Favourably the at least one attachment tab is provided with an adhesive in order to releasably attach the shield to the infant's skin. Preferably the adhesive is a light adhesive. More preferably the adhesive is single-use and of low viscosity, in order to avoid damage or discomfort to the infant. The at least one attachment tab is ideally breathable, latex-free and hypoallergenic, such as a paper tape that is gentle to the skin yet adheres well and leaves minimal adhesive residue upon removal. The at least one attachment tab may be fabricated from a microporous material.

Secondary tabs or flaps may be provided in preferred embodiments which allow the parent to lift the flaps and peel back the attachment tabs. This aids removal of the shield from the user.

Favourably the shield varies in depth from the front end to the rear end, such that the form of the sides is curved or truncated in order that the form is largely inclined. Preferably in addition the form may include an indentation in this incline such to provide an arcuate form.

Preferably therefore the form is such that the shield may be placed over the infant's genital area with the form following the curve of the infant's body with the incline of the form meaning that the shield is deeper at the rear end, towards the infant's legs.

The shield is furthermore preferably shaped so they can easily sit inside one another. When bought, the shields may come stacked.

The shield may comprise a detachable portion forming the base. At least one of the base and side walls may be washable.

Favourably each of the base and side walls further comprise an outer layer, the outer layer being substantially water resistant.

The shield may further include a sterile absorbent pad suitable for collecting a urine sample. The sterile absorbent pad may be removable from the shield.

The shield may further accommodate a layer which acts as an assay strip. Such a layer may be removable. An assay strip may be incorporated into the base or sides of the shield. An assay strip could be used to detect presence of relevant substances: for example: haemoglobin, nitrate (produced by bacteria in a urinary tract infection), protein, glucose, blood, and ketones. Favourably, at least part of the assay strip may be configured to change colour in response to a positive detection.

A further aspect of the invention provides a device for collecting a urine sample from a user comprising an elongate base and side walls connected to the base, at least one attachment tab for releasably attaching the device to the user's skin and at least one absorbent sterile pad.

The absorbent sterile pad may form part of an absorbent element, the absorbent element supported by at least one of the said side walls and base.

Preferably the device has an hourglass shaped cross section. This makes the device more comfortable for the user.

Preferably the at least one attachment tab is provided with an adhesive in order to releasably attach the shield to the user's skin.

The device may also include an assay strip. The assay strip may form part of an absorbent element, the absorbent element supported by at least one of the said side walls and base. Alternatively, the assay strip may be configured as a layer within at least one of the base and side walls of the device.

Favourably an assay strip is configured to detect a substance selected from the group comprising: haemoglobin, nitrate, protein, glucose, blood, and ketones. More favourably, at least part of the assay strip may be configured to change colour in response to a positive detection.

Preferably the device is sterile.

A further aspect of the invention provides a method of collecting a urine sample from an individual comprising the steps of (a) attaching a shield as hereinbefore defined in to the individual's genital area; (b) awaiting urination of the individual; and (c) removing the shield.

Favourably the method further comprises the additional step of (d) removing the assay strip. The assay strip or the shield containing the assay strip could then be sent away for testing.

Preferably, when used for collecting a urine sample, the shield would be provided in a sterile condition.

The shield's shape and secured position on the infant's body will reduce the likelihood of contamination which is associated with current pad or bag urine collection methods. Such contamination is liable to yield false positive results.

The shield of the invention provides an improved urine shield for shielding carers from urination events during nappy or diaper changes or during medical procedures. The shield of the invention can also provide a more convenient method for collection of urine samples than the current clean-catch method as it can be attached in place, without the need for the parent/carer to be waiting for the child to urinate.

### Brief Description of Figures

Figure 1 shows a front isometric view from above of a preferred embodiment of the shield;

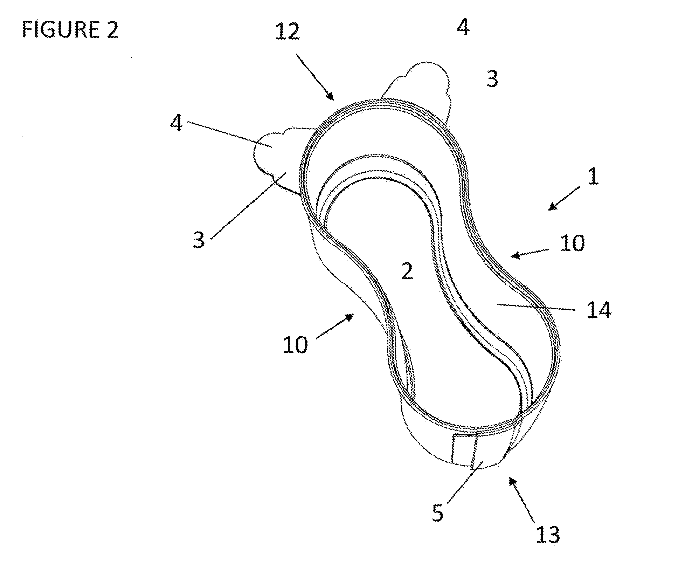

Figure 2 shows an isometric view of the shield of Figure 1 from underneath;
Figure 3 shows an isometric view of several shields of Figure 1 stacked together;
Figure 4 shows a side view of the shield of Figure 1;
Figure 5 shows the shield of Figure 1 in use, adhered to an infant's genital area;
Figure 6 shows a plan view of an alternative embodiment of the shield including an assay strip;
Figure 7 shows an isometric view of an alternative embodiment of the shield; and
Figure 8 shows a plan view of the embodiment of Figure 7.

### Detailed Description of Preferred Embodiments of the Invention

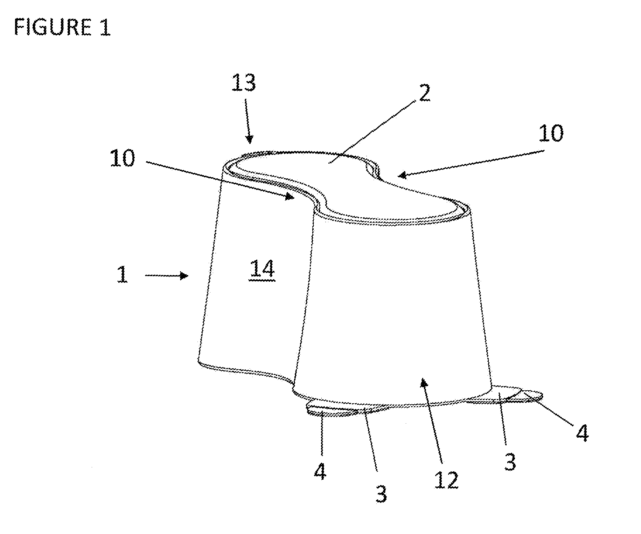

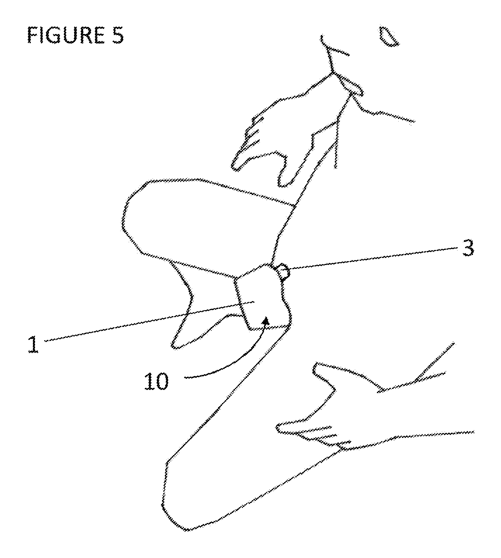

A urine shield 1 according to a preferred embodiment of the invention is shown in Figure 1. The shield comprises a base 2 and side walls 14 which extend continuously around the base 2. The base 2 is elongate in shape and has a narrow portion in the centre such that both the front end 12 and the rear end 13 are wider than the middle portion, providing the shield with curved wall portions forming an indentation 10. The shield 1 has an hourglass type shape in plan view, and in this embodiment the shape of the shield is symmetrical about the middle portion, or hourglass waist, in plan view. The indentation 10 in the wall profile allows the infant greater freedom of movement, reducing the irritation. Ideally this indentation 10 is situated in use centrally, such as where the infant's legs are closest to one another (as shown in Figure 5).

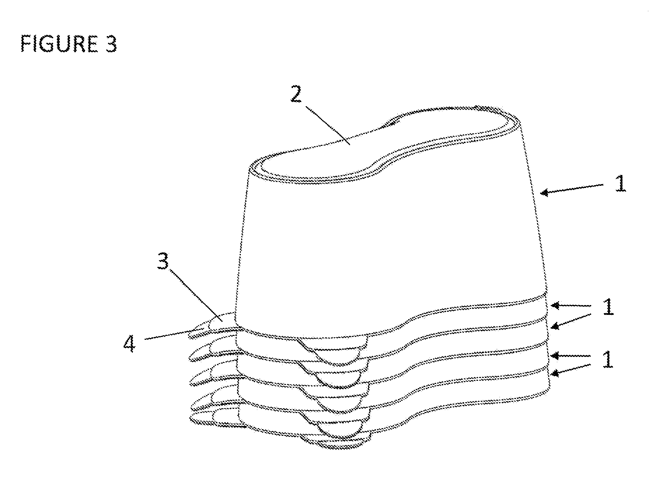

In use the shield may be provided in a stack or multiple packet (as shown in Figure 3), where the carer may take a shield from the packet.

The shield 1 is provided with attachment tabs 3 located at one end of the shield. The attachment tabs 3 are provided with a light adhesive. Preferably the tabs 3 are located at the front end 12 of the shield 1 and are attached to the infant's lower stomach area as shown in Figure 5.

The attachment tabs 3 are ideally provided with a backing (not shown) to prevent contact or loss of adhesiveness. In use therefore the carer must remove the adhesive's backing, exposing the adhesive layer, before the tabs 3 can be used for attachment purposes.

The carer then places the shield on the infant (as shown in Figure 5). The shield may have a pattern, with this pattern being reactive to urine in some embodiments.

Ideally in such embodiments a wetness indicator is disposed between a translucent cover member and an absorbent member on the continuous side and base of the shield. This flexible pH-change/colour-change wetness indicator is typically an internal exterior coating and is visible through the cover member, ideally retaining definition of the exterior coating when wetted; for instance, by urine.

Such a coating may comprise a solid-solid mixture (e.g., solution) of a pH-change/colour-change type material dispersed in a matrix. Therefore ideally if the infant urinates when the shield is in use, colour would appear within the pattern, thus alerting the carer.

Such an arrangement allows a carer to be confident to leave the shield in place and be aware when to change it. This also allows the infant's skin to breathe.

In the embodiment illustrated the attachment tabs 3 are also provided with flaps 4 to aid removal of the shield 1 from the infant. To remove the shield the carer would grasp the flaps 4 and pull the shield 1 gently away from the infant. In the pictured embodiment the flaps 4 are of a semi-circular form allowing a user/carer to grasp the flap between thumb and forefinger, with the force spreading out across the surface of the flap and acting upon the tab 3 equally at the perimeter of its adhesiveness.

Ideally therefore in the majority of embodiments the flaps 4 are sufficiently close to the tabs 3 and connected such that flexure of the flaps 4 causes flexure of the tabs 3 in tandem. This aids removal of the shield from the infant ideally by leading to a gradual removal of the tab(s).

In some embodiments the shield may be provided in two parts with the base 2 placed inside the continuous side walls 14. In the pictured embodiment a seam 5 is shown where the side walls 14 may be detached. In some embodiments the base 2 and side walls 14 may be detached and one or both may be washable. In addition portions may be removable from the shield in order to wash them.

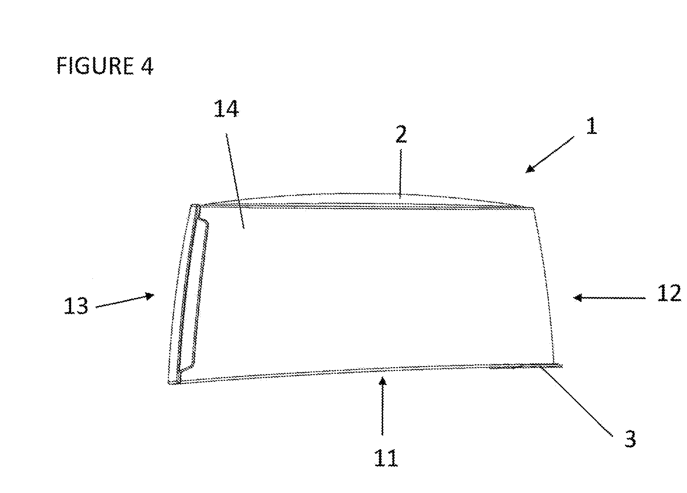

Figure 4 illustrates a side view of the shield 1. It can be seen from this view that the rear end 13 of the shield 1 is slightly deeper than the front end 12 of the shield, giving the shield a slightly curved side profile 11. This slightly curved profile to the form of the shield serves to fit the shield to the infant's body. In further embodiments this curve may be more acute and the variation in depth from one end 12 to the other end 13. This is towards placing the less deep end on the stomach of the infant and the deepest end is placed between the legs of the infant in use, such that any urination will be contained, in whichever direction it is aimed.

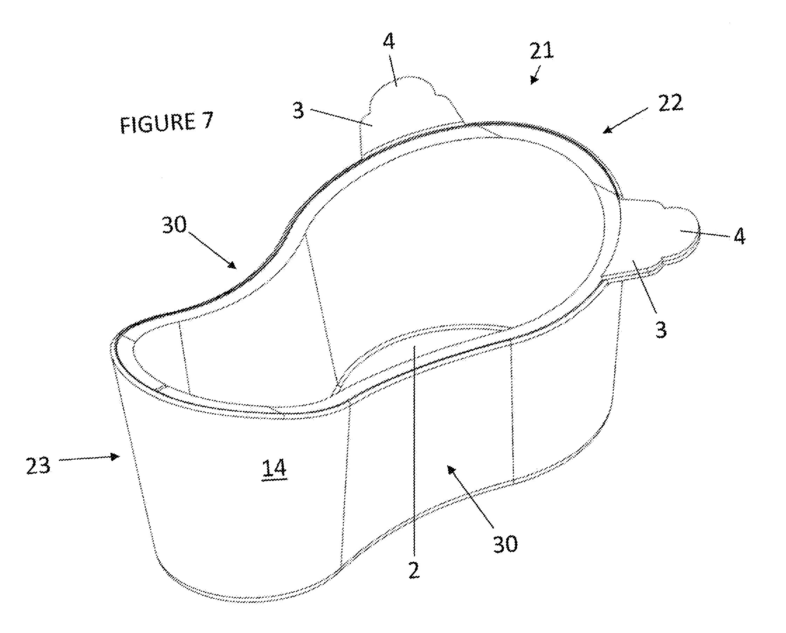

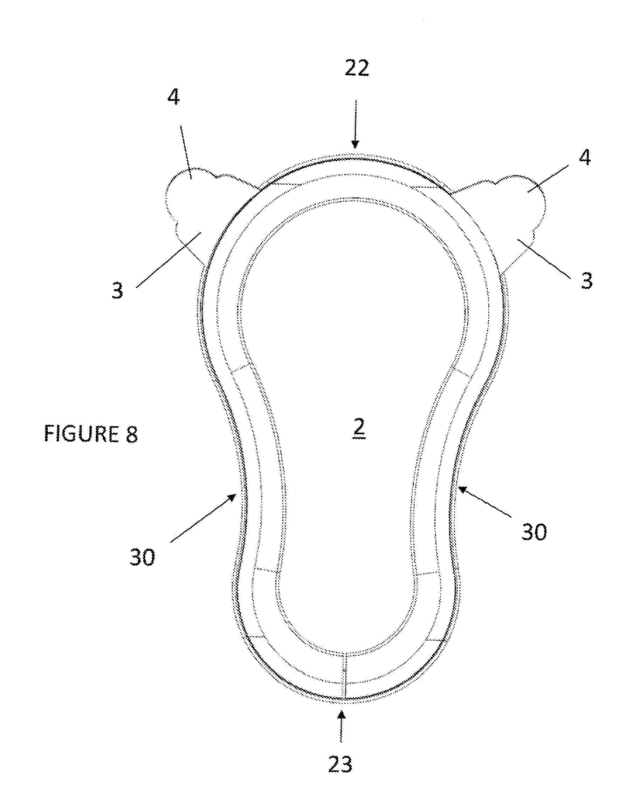

A urine shield 21 according to an alternative embodiment of the invention is shown in Figures 7 and 8. As with the previously described embodiment, the shield comprises a base 2 and side walls 14 which extend continuously around the base 2. The base 2 is elongate in shape and has a narrow portion in the centre such that both the front end 22 and the rear end 23 are wider than the middle portion, providing the shield with curved wall portions 30. The shield 1 has an hourglass type shape in plan view, as illustrated in Figure 8. In this embodiment of the invention the front end 22 is wider than the rear end 23, such that the shape is asymmetrical about the hourglass waist. The indentation 30 in the wall profile allows the infant greater freedom of movement, reducing the irritation. Ideally this indentation 30 is situated in use centrally, such as where the infant's legs are closest to one another.

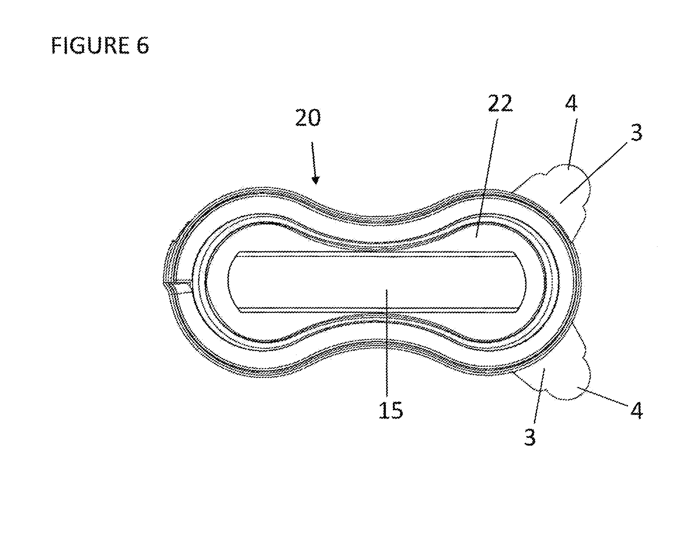

Figure 6 illustrates a plan view of an embodiment of the invention which includes an assay strip 15 inside the shield 20. In this example the assay strip 15 is located on the base 2, inside the shield 20.

This embodiment of the invention could be used to collect a urine sample. Once saturated with urine the assay strip may be removed and sent away for urinalysis. Alternatively, the entire shield 20 may be sent away for urinalysis without prior removal of the assay strip. In an alternative embodiment the assay strip may be impregnated with a chemical that allows instant detection of certain substances such as haemoglobin, nitrate, protein, glucose, blood, or ketones. This may be achieved, for example, by configuring the assay strip 15 to change colour in response to a positive result.

If used to collect a urine sample, then the shield would preferably be provided in a sterile condition, inside a sterile package.

The invention has been described by way of examples only and it will be appreciated that variation may be made to the above-mentioned embodiments without departing from the scope of invention.

With respect to the above description then, it is to be realised that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

## Claims

1. A urine shield (1, 20, 21) comprising an elongate base (2) and side walls (14), the side walls (14) have a lower edge and an upper edge, and wherein the side walls (14) are connected to the base at the lower edge , the upper edge distal from the base (2) and for engagement with a user's skin, at least one of said side walls (14) and base (2) being absorbent or supporting an absorbent element, the shield further comprising at least one attachment tab (3) for releasably attaching the shield to a the user's skin, wherein the shield has an hourglass shaped cross-section, **characterised in that** the side walls extend continuously around the base and wherein with the upper edge of the side walls in engagement with the user's skin, the base is spaced apart from the user's genital area.

2. A urine shield according to Claim 1, wherein the hourglass shaped cross-section includes a waist (10, 30) and wherein said hourglass shaped cross-section is either symmetrical or asymmetrical about the waist.

3. A urine shield according to any preceding claim, wherein both the side walls (14) and the base (2) are absorbent.

4. A urine shield according to any preceding claim, wherein the at least one attachment tab (3) is provided with an adhesive.

5. A urine shield according to any preceding claim with a detachable portion forming the base (2).

6. A urine shield according to any preceding claim, wherein the shield (1, 20, 21) is stackable with respect to another shield (1, 20, 21), wherein the side walls (14) are configured such that shields (1, 20, 21) are stackable one within another.

7. A urine shield according to any preceding claim, wherein the at least one attachment tab (3) is attached to the side walls (14).

8. A urine shield according to any preceding claim, wherein at least one of the base (2) and sides (14) is provided with a urine indicator that changes colour when exposed to urine.

9. A urine shield according to any preceding claim, wherein each of the base (2) and side walls (14) further comprise an outer layer, the outer layer being water resistant.

10. A urine shield according to any preceding claim, further comprising a sterile absorbent pad.

11. A urine shield according to claim 10, wherein the sterile absorbent pad is removable.

12. A urine shield according to any preceding claim, further comprising an assay strip (15).

13. A urine shield according to Claim 12, wherein the assay strip (15) is configured to detect a substance selected from the group comprising haemoglobin, nitrate, protein, glucose, blood and ketones.

14. A urine shield according to Claim 12 or 13, wherein part of the assay strip (15) is configured to change colour in response to a positive detection.

15. A urine shield according to any preceding claim, the shield configured as a device for collecting a urine sample.

## Patentansprüche

1. Urinschutzvorrichtung (1, 20, 21), die eine längliche Basis (2) und Seitenwände (14) umfasst, wobei die Seitenwände (14) eine Unterkante und eine Oberkante aufweisen und wobei die Seitenwände (14) an der Unterkante mit der Basis verbunden sind, wobei die Oberkante von der Basis (2) entfernt ist und zu einer Bindung mit der Haut eines Benutzers ist, wobei die Seitenwände (14) und/oder die Basis (2) absorptionsfähig sind oder ein absorptionsfähiges Element tragen, wobei die Schutzvorrichtung weiterhin mindestens eine Anbringungslasche (3) zum lösbaren Anbringen der Schutzvorrichtung an der Haut des Benutzers umfasst, wobei die Schutzvorrichtung einen sanduhrförmigen Querschnitt hat, **dadurch gekennzeichnet, dass** die Seitenwände sich durchgängig um die Basis herum erstrecken und wobei, wenn die Oberkante der Seitenwände in einer Bindung mit der Haut des Benutzers ist, die Basis von dem Genitalbereich des Benutzers beabstandet ist.

2. Urinschutzvorrichtung nach Anspruch 1, wobei der sanduhrförmige Querschnitt eine Taille (10, 30) beinhaltet und wobei der sanduhrförmige Querschnitt entweder symmetrisch oder asymmetrisch um die Taille ist.

3. Urinschutzvorrichtung nach einem vorhergehenden Anspruch, wobei sowohl die Seitenwände (14) als auch die Basis (2) absorptionsfähig sind.

4. Urinschutzvorrichtung nach einem vorhergehenden Anspruch, wobei die mindestens eine Anbringungslasche (3) mit einem Klebstoff versehen ist.

5. Urinschutzvorrichtung nach einem vorhergehenden Anspruch mit einem abnehmbaren Teil, der die Basis (2) bildet.

6. Urinschutzvorrichtung nach einem vorhergehenden Anspruch, wobei die Schutzvorrichtung (1, 20, 21) stapelbar in Bezug auf eine andere Schutzvorrichtung (1, 20, 21) ist, wobei die Seitenwände (14) derart konfiguriert sind, dass Schutzvorrichtungen (1, 20, 21) ineinander stapelbar sind.

7. Urinschutzvorrichtung nach einem vorhergehenden Anspruch, wobei die mindestens eine Anbringungslasche (3) an den Seitenwänden (14) angebracht ist.

8. Urinschutzvorrichtung nach einem vorhergehenden Anspruch, wobei die Basis (2) und/oder die Seiten (14) mit einer Urinanzeige versehen sind, die ihre Farbe ändert, wenn sie Urin ausgesetzt wird.

9. Urinschutzvorrichtung nach einem vorhergehenden Anspruch, wobei die Basis (2) und die Seitenwände (14) jeweils weiterhin eine Außenschicht umfassen, wobei die Außenschicht wasserfest ist.

10. Urinschutzvorrichtung nach einem vorhergehenden Anspruch, die weiterhin ein steriles absorptionsfähiges Pad umfasst.

11. Urinschutzvorrichtung nach Anspruch 10, wobei das sterile absorptionsfähige Pad entfernbar ist.

12. Urinschutzvorrichtung nach einem vorhergehenden Anspruch, die weiterhin einen Teststreifen (15) umfasst.

13. Urinschutzvorrichtung nach Anspruch 12, wobei der Teststreifen (15) dazu konfiguriert ist, eine Substanz zu erkennen, die aus der Gruppe bestehend aus Hämoglobin, Nitrat, Protein, Glukose, Blut und Ketonen ausgewählt ist.

14. Urinschutzvorrichtung nach Anspruch 12 oder 13, wobei ein Teil des Teststreifens (15) dazu konfiguriert ist, als Reaktion auf eine positive Erkennung seine Farbe zu ändern.

15. Urinschutzvorrichtung nach einem vorhergehenden Anspruch, wobei die Schutzvorrichtung als ein Hilfsmittel zum Nehmen einer Urinprobe konfiguriert ist.

## Revendications

1. Une protection contre l'urine (1, 20, 21) comprenant une base de forme allongée (2) et des parois latérales (14), les parois latérales (14) ont un bord inférieur et un bord supérieur, et dans laquelle les parois latérales (14) sont reliées à la base au bord inférieur, au bord supérieur distal de la base (2) et pour s'engager avec la peau d'un utilisateur, au moins une desdites parois latérales (14) et de ladite base (2) étant absorbantes ou soutenant un élément absorbant, la protection comprenant en outre au moins une languette de fixation (3) pour attacher la protection de façon amovible à la peau de l'utilisateur, dans laquelle la protection a une section transversale en forme de sablier, **caractérisée en ce que** les parois latérales s'étendent de manière continue autour de la base et dans laquelle avec le bord supérieur des parois latérales engagé avec la peau de l'utilisateur, la base est située à une certaine distance de la zone génitale de l'utilisateur.

2. Une protection contre l'urine selon la revendication 1, dans laquelle la section transversale en forme de sablier inclut une ceinture (10, 30) et dans laquelle ladite section transversale en forme de sablier est soit symétrique soit asymétrique par rapport à la ceinture.

3. Une protection contre l'urine selon l'une quelconque des revendications précédentes, dans laquelle les deux parois latérales (14) et la base (2) sont absorbantes.

4. Une protection contre l'urine selon l'une quelconque des revendications précédentes, dans laquelle la ou les languettes de fixation (3) sont munies d'un adhésif.

5. Une protection contre l'urine selon l'une quelconque des revendications précédentes avec une partie détachable formant la base (2).

6. Une protection contre l'urine selon l'une quelconque des revendications précédentes, dans laquelle la protection (1, 20, 21) est empilable avec une autre protection (1, 20, 21), dans laquelle les parois latérales (14) sont configurées de telle sorte que les protections (1, 20, 21) sont empilables les unes avec les autres.

7. Une protection contre l'urine selon l'une quelconque des revendications précédentes, dans laquelle la ou les languettes de fixation (3) sont attachées aux parois latérales (14).

8. Une protection contre l'urine selon l'une quelconque des revendications précédentes, dans laquelle au moins soit la base (2) soit l'une des parois latérales (14) est munie d'un indicateur d'urine qui change de couleur quand il est exposé à l'urine.

9. Une protection contre l'urine selon l'une quelconque des revendications précédentes, dans laquelle la base (2) et les parois latérales (14) comprennent en outre chacune une couche extérieure, la couche extérieure étant sensiblement résistante à l'eau.

10. Une protection contre l'urine selon l'une quelconque des revendications précédentes, comprenant en outre un tampon absorbant stérile.

11. Une protection contre l'urine selon la revendication 10, dans laquelle le tampon absorbant stérile est amovible.

12. Une protection contre l'urine selon l'une quelconque des revendications précédentes, comprenant en outre une bandelette d'analyse (15).

13. Une protection contre l'urine selon la revendication 12, dans laquelle la bandelette d'analyse (15) est configurée pour détecter une substance sélectionnée dans le groupe comprenant l'hémoglobine, les nitrates, les protéines, le glucose, le sang et les cétones.

14. Une protection contre l'urine selon la revendication 12 ou 13, dans laquelle une partie de la bandelette d'analyse (15) est configurée pour changer de couleur en réaction à une détection positive.

15. Une protection contre l'urine selon l'une quelconque des revendications précédentes, la protection étant configurée pour être un dispositif permettant de recueillir un échantillon d'urine.
